# EUROPÄISCHE PATENTSCHRIFT

(11) **EP 2 089 086 B1**
(45) Veröffentlichungstag und Bekanntmachung des Hinweises auf die Patenterteilung: **07.05.2014**
(21) Anmeldenummer: 07817766.4
(22) Anmeldetag: 03.11.2007
(51) Int. Cl.: A61M 16/08

(54) **Atemschlauch-Anschlussvorrichtung**
Respiratory tube connecting device
Dispositif de raccordement de flexible respiratoire

(30) Priorität: 14.11.2006 DE 102006053857
(43) Veröffentlichungstag der Anmeldung: 19.08.2009
(60) Teilanmeldung: 13189588.0
(73) Patentinhaber: Dräger Medical GmbH, 23558 Lübeck (DE)
(72) Erfinder: TAPPEHORN, Ludger, 23560 Lübeck (DE); WOTHA, Gerd, 23626 Warnsdorf (DE); SCHERMEIER, Olaf, 23560 Lübeck (DE); ABRAHAM, Klaus, 23560 Lübeck (DE)
(74) Vertreter: UEXKÜLL & STOLBERG
(86) Internationale Anmeldenummer: PCT/DE2007/001975
(87) Internationale Veröffentlichungsnummer: WO 2008/058506

(56) Entgegenhaltungen:
- EP-A- 1 138 340
- EP-A- 1 520 599
- DE-A1-102005 001 247
- US-B1- 6 484 724

## Beschreibung

Die Erfindung betrifft eine Atemschlauch - Anschlussvorrichtung zur Verbindung eines Atemschlauches mit einer Atemvorrichtung mit den Merkmalen von Anspruch 1.

Atemschlauch - Anschlussvorrichtungen dienen in bekannter Weise der Verbindung eines Atemschlauches oder eines Atemschlauchsystems, im Folgenden der Einfachheit halber mit Atemschlauch bezeichnet, mit einer Atemvorrichtung. Eine Atemvorrichtung kann ein Anästhesiegerät oder ein Beatmungsgerät umfassen. Die Atemschlauch - Anschlussvorrichtungen unterscheiden sich hinsichtlich des Durchmessers eines Atemgasdurch-gangskanals. So weisen beispielsweise Atemschlauch - Anschlussvorrichtungen für den neonatalen Einsatz geringere Durchmesser auf, im Vergleich zu den Atemschlauch - Anschlussvorrichtungen zur Verbindung eines Atemschlauches an eine Atemvorrichtung für Erwachsene. Die Atemschlauch-Anschlussvorrichtungen verfügen dabei entweder über einen männlichen oder einen weiblichen Anschluss. Ein dazu komplementärer Anschluss ist jeweils an der Atemvorrichtung zu finden. Üblicherweise sind Atemschlauch - Anschlussvorrichtungen für eine Verbindung mit einer neonatalen Atemvorrichtung mit einem weiblichen Anschluss ausgeführt, wohingegen Atemschlauch - Anschlussvorrichtungen für eine Verbindung mit einer Atemvorrichtung für Erwachsene mit einem männlichen oder weiblichen Anschluss ausgeführt sein können. Demgegenüber sind die Anschlüsse an der neonatalen Atemvorrichtung üblicherweise als männlicher Anschluss und an der Atemvorrichtung für Erwachsene als Kombination von männlichem und weiblichem Anschluss ausgeführt.

Es existiert für die Atemschlauch - Anschlussvorrichtungen ein gängiger Standard von 11, 15, und 22 Millimeter Durchmesser des Atemgasdurchgangskanals. Dabei weisen Atemschlauch - Anschlussvorrichtungen für den neonatalen Einsatz einen Durchmesser des Atemgasdurchgangskanals von 11 Millimetern auf, wohingegen Atemschlauch - Anschlussvorrichtungen für Atemvorrichtungen für Erwachsene einen Durchmesser des Atemgasdurchgangskanals von 15 oder 22 Millimetern aufweisen können.

Die Atemvorrichtungen für Erwachsene verfügen im Allgemeinen über einen Atemmodus für Neonaten. Soll nun mit einer Atemvorrichtung für Erwachsene ein neonataler Patient beatmet werden, ist es wünschenswert, dass sich die Atemschlauch - Anschlussvorrichtung des neonatalen Atemschlauches, die für einen Anschluss an eine neonatale Atemvorrichtung konzipiert ist, sowohl mit der neonatalen Atemvorrichtung als auch mit der Atemvorrichtung für Erwachsene verbinden lässt. Darüber hinaus sollte die Atemschlauch - Anschlussvorrichtung einfach und kostengünstig herzustellen sein.

Aus EP 1 520 599 A1 ist eine Verbindungseinrichtung zur Verbindung von zwei Atemschläuchen miteinander bekannt, von denen einer zur Verwendung in unmittelbarer Nähe des Patienten und zum Anschluss an eine Nasenkanüle vorgesehen ist. Die Verbindungseinrichtung weist zwei ineinandersteckbare Verbindungselemente auf, die jeweils zur Verbindung mit einem der Atemschläuche vorgesehen sind und in der ineinandergesteckten Position durch eine Hülse gesichert werden, die um den Verbindungsbereich zwischen den beiden Verbindungselementen herum angeordnet werden kann. Die Verbindungseinrichtung ist dazu vorgesehen und ausgestaltet, um an dem Patienten befestigt zu werden.

DE 10 2005 001 247 A1 offenbart eine Vorrichtung, die ausgebildet ist, um zwischen einer Schlauchleitung und einer Atemmaske angeordnet zu werden und maskennah vom die Atemmaske tragenden Patienten exhaliertes Atemgas aus dem Atemgasleitungssystem abzuleiten. Dazu weist sie einen auf einer Seite mit einer Schlauchleitung und auf einer anderen Seite mit einer Atemmaske gekoppelten Anschlussabschnitt auf, um den herum in einem mittleren Bereich ein verdrehbares Ringelement angeordnet ist. Zwischen dem Anschlussabschnitt und dem Ringelement sind Ableitungskanäle ausgebildet, durch die Gas aus dem Inneren des Anschlussabschnitts nach außen entweichen kann. Durch Verdrehen des Ringelements kann der Querschnitt der Ableitungskanäle verändert werden.

Eine ähnliche Vorrichtung zur Ableitung von Atemgas aus einem Atemgasleitungssystem ist aus der EP 1 138 340 A2 bekannt. Sie ist angepasst, um an dem von einem Beatmungsgerät abgewandten Ende einer Schlauchleitung mit einer Atemmaske gekoppelt zu werden. Zur Ableitung des exhalierten verbrauchten Atemgases sind in der Ableitungsvorrichtung mehrere Kanäle ausgebildet, durch die das Gas nach außen gelangen kann.

US 6,484,724 B1 offenbart eine Atemschlauch-Anschlussvorrichtung gemäß dem Oberbegriff des Anspruchs 1.

Die Aufgabe der Erfindung besteht demnach in der Bereitstellung einer einfach herzustellenden Atemschlauch - Anschlussvorrichtung, die das vorstehend beschriebene Problem beseitigt, sowie in einem Verfahren zur Herstellung einer derartigen Atemschlauch - Anschlussvorrichtung.

Diese Aufgabe wird erfindungsgemäß durch eine Atemschlauch - Anschlussvorrichtung mit den Merkmalen des Anspruchs 1 gelöst. Die Idee der Erfindung ist es, einen Grundkörper mit einem, dem Durchmesser des neonatalen Atemschlauches entsprechenden Atemgasdurchgangskanal und einen, in den Abmessungen dem Anschluss an die jeweilige Atemvorrichtung für Erwachsene entsprechend ausgebildeten Hüllkörper bereitzustellen, wobei der Grundkörper mit dem, für die entsprechende Atemvorrichtung für Erwachsene, vorgesehenen Hüllkörper kombinierbar ist.

Die Idee der Erfindung ist es, einen Grundkörper mit einem, dem Durchmesser des neonatalen Atemschlauches entsprechenden Atemgasdurchgangskanal und einen, in den Abmessungen dem Anschluss an die jeweilige Atemvorrichtung für Erwachsene entsprechend ausgebildeten Hüllkörper bereitzustellen, wobei der Grundkörper mit dem, für die entsprechende Atemvorrichtung für Erwachsene, vorgesehenen Hüllkörper kombinierbar ist und in der Kombination der Grundkörper und der Hüllkörper in formschlüssigen Eingriff gebracht sind, so dass ein Anschluss eines neonatalen Atemschlauches mittels der erfindungsgemäßen Atemschlauch - Anschlussvorrichtung sowohl an eine neonatale Atemvorrichtung, als auch an eine Atemvorrichtung für Erwachsene erfolgen kann.

Vorteilhafte Ausführungen und Weiterbildungen der Erfindung sind in den Unteransprüchen angegeben.

Die mit der Erfindung erzielten Vorteile bestehen insbesondere darin, dass die erfindungsgemäße Atemschlauch - Anschlussvorrichtung sehr kostengünstig herstellbar ist. Es ist dabei ein Grundkörper herstellbar, der mit einem gemäß der jeweiligen Atemvorrichtung für Erwachsene entsprechenden Hüllkörper derart kombinierbar und in formschlüssigen Eingriff gebracht ist, dass die Atemschlauch - Anschlussvorrichtung sowohl an die neonatale Atemvorrichtung als auch an die Atemvorrichtung für Erwachsene anschließbar ist. Ein jeweils gleicher Grundkörper kann also mit einem Hüllkörper, der einen zum Anschluss an die entsprechende Atemvorrichtung für Erwachsene erforderlichen Durchmesser aufweist, kombiniert werden. Die erfindungsgemäße Atemschlauch - Anschlussvorrichtung ermöglicht somit eine Verbindung eines Atemschlauches sowohl mit einer ersten, als auch mit einer zweiten Atemvorrichtung, wobei die erste und die zweite Atemvorrichtung eine unterschiedliche Anschlussstruktur aufweist. Die erste Atemvorrichtung ist dabei eine neonatale Atemvorrichtung und die zweite Atemvorrichtung eine Atemvorrichtung für Erwachsene. Die Atemvorrichtung für Erwachsene weist zudem einen Atemmodus zur Beatmung von Neonaten auf.

Der Aufbau der erfindungsgemäßen Atemschlauch - Anschlussvorrichtung ist derart gestaltet, dass sowohl die Außenfläche des Grundkörpers als auch die Innenwandung des Hüllkörpers vorzugsweise zylindrisch ausgebildet sind. Diese Formgestaltung ermöglicht eine stabile Verbindung von Grund- und Hüllkörper. Ein Formschluss von Grund- und Hüllkörper erfolgt vorzugsweise durch einen Eingriff einer an der Außenfläche des Grundkörpers vorgesehenen Umfangswulst in eine komplementäre, an der Innenwandung des Hüllkörpers vorgesehene Umfangsnut. Um nach dem formschlüssigen Eingreifen von Grund- und Hüllkörper ein Verdrehen des Hüllkörpers in Bezug zu dem Grundkörper zu verhindern, insbesondere bei einem Verbinden oder Lösen der Atemschlauch - Anschlussvorrichtung mit oder von der Atemvorrichtung, sind in einer weiteren Ausführung der erfindungsgemäßen Vorrichtung Mittel vorgesehen, die ein Verdrehen verhindern. Vorzugsweise weist dafür der Grundkörper eine senkrecht zu der Außenfläche des Grundkörpers gerichtete Nase auf und der Hüllkörper eine komplementäre, senkrecht zu seiner Innenwandung gerichtete Aussparung. Die Nase der Außenfläche des Grundkörpers tritt bei Formschluss mit der Aussparung der Innenwandung des Hüllkörpers in Eingriff. Ein Material des Grund- und Hüllkörpers besteht vorzugsweise aus Polypropylen. Polypropylen ist in bekannter Weise ein elastisches Material, was den Vorgang des Formschlusses von Grund- und Hüllkörpers begünstigt. Zudem verläuft für eine optimale Konnektierung der Atemschlauch - Anschlussvorrichtung mit einer Atemvorrichtung der zu einer Atemvorrichtung gerichtete Teil des Atemgasdurchgangskanals des Grundkörpers und die Außenfläche des Hüllkörpers konusförmig.

Der Transponder ist in vorteilhafter Weise zwischen dem Grundkörper und dem Hüllkörper vorgesehen. Transponder weisen eine hohe Wärmeempfindlichkeit auf. Ein Einbringen in den Grund- oder Hüllkörper während einer Herstellung des Grund- oder Hüllkörpers im Spritzgußverfahren ist ohne eine Zerstörung seiner Funktionen nahezu unmöglich. Eine Positionierung des Transponders auf der Außenfläche der Atemschlauch - Anschlussvorrichtung, beispielsweise durch Aufkleben, kann einerseits Beschädigungen des Transponders beim Verbinden der Atemschlauch - Anschlussvorrichtung mit der Atemvorrichtung verursachen und andererseits zu einer Undichtheit der Verbindung Atemvorrichtung / Patient führen.

Der erfindungsgemäße Aufbau der Atemschlauch - Anschlussvorrichtung ermöglicht eine Positionierung des Transponders zwischen der Außenfläche des Grundkörpers und der Innenwandung des Hüllkörpers, so dass der Transponder von dem ihn umgebenden Hüllkörper der Atemschlauch - Anschlussvorrichtung vor Beschädigungen geschützt wird.

Auf der Außenfläche des Grundkörpers sind vorzugsweise Hilfsmittel zur Positionierung des Transponders auf dem Grundkörper vorgesehen. Beispielsweise kann die an der Außenfläche des Grundkörpers vorgesehene Umfangswulst als Positionierungshilfe beim Aufbringen des Transponders auf den Grundkörper vorgesehen werden. Der Transponder ist bevorzugt an einer zu der Atemvorrichtung weisenden Seite des Grundkörpers angebracht. Damit können auch passive Transponder mit einer stark eingeschränkten Reichweite zum Einsatz kommen. Dies kann beispielsweise ein RFID Transponder sein, der eine Reichweite von zirka 18 Millimetern aufweist.

Der Transponder wird vorzugsweise auf die Außenfläche des Grundkörpers mittels eines geeigneten Klebeverfahrens aufgebracht. Klebeverfahren zum Aufbringen von Transpondern auf der Oberfläche eines bestimmten Teils sind aus dem Stand der Technik bekannt, daher wird an dieser Stelle nicht näher darauf eingegangen. Es kann jedoch auch in einem bevorzugten Verfahrensschritt kurz vor Beendigung oder nach der Herstellung des Grundkörpers durch ein Spritzgussverfahren vorgesehen sein, den Transponder auf den Grundkörper aufzubringen, indem der Grundkörper in einem Spritzgusswerkzeug vorzugsweise nach dessen Herstellung vorerst verbleibt und der Transponder mittels einer geeigneten Spritzgusstechnik auf die Außenfläche des Grundkörpers aufgebracht wird, ohne den Transponder dabei vollständig mit Spritzgussmaterial zu umgeben.

In einer besonders bevorzugten Ausführung der Atemschlauch - Anschlussvorrichtung ist der Transponder mit einer Funktion zur Speicherung der Daten ausgestattet. Der Transponder ermöglicht durch die Speicherfunktion von Daten in vorteilhafter Weise eine Ablage von Atemparametern. Als Atemparameter können beispielsweise eine Atemfrequenz, ein Atemhub, ein Atemdruck und / oder eine Form der Beatmung hinterlegt werden. Auch sind Kenngrössen für eine Identifikation des Atemschlauches hinterlegbar. Der Atemschlauch verbleibt bei einem Wechsel der Atemvorrichtung, beispielsweise aufgrund eines Wechsels des klinischen Bereiches des Patienten, bei dem Patienten. Die in dem Transponder hinterlegten Daten sind somit mit dem Atemschlauch verfügbar und ermöglichen bei einem Anschluss des Atemschlauches an eine andere Atemvorrichtung eine automatische oder semiautomatische Einstellung der erforderlichen Atemparameter an dieser Atemvorrichtung. Somit lassen sich auf einfache Weise Einstellparameter einer ersten Atemvorrichtung auf eine zweite Atemvorrichtung übertragen. Des Weiteren können Daten über verbotene Parameter von weiteren Geräten in dem Transponder der Atemschlauch - Anschlussvorrichtung hinterlegt sein. Beispielsweise kann bei Anschluss des neonatalen Atemschlauches an eine Atemvorrichtung für Erwachsene nach einer Erkennung des neonatalen Atemschlauches ein Atemmodus für Erwachsene blockiert werden.

Darüber hinaus ermöglicht der Transponder der erfindungsgemässen Vorrichtung eine Erfassung und Ablage von personengebundenen Daten wie beispielsweise eine Patentenidentifikationsnummer. Mittels einer Patientenidentifikationsnummer kann sich eine, an ein Kliniknetzwerk angeschlossene Atemvorrichtung auf die für den entsprechenden Patienten erforderlichen Atemparameter einstellen.

In einer weiteren Gestaltung der erfindungsgemässen Atemschlauch-Anschlussvorrichtung werden Kenngrößen für eine Gewährleistung einer einwandfreien Funktion des Atemschlauches übertragen. Beispielsweise kann eine Lebensdauer des Atemschlauches auf dem Transponder hinterlegt werden und nach einem Zeitablauf, beispielsweise einer für eine Gewährleistung einer einwandfreien Funktion des Atemschlauches ermittelte und festgelegte Zeit, eine entsprechende Information dem Anwender übermittelt werden.

Ausführungsbeispiele der Erfindung sind in den Figuren 1 bis 4 beschrieben.

Dabei zeigen:
- Fig. 1: eine vereinfachte Schnittdarstellung der erfindungsgemässen Atemschlauch - Anschlussvorrichtung in einer ersten Ausführung,
- Fig. 2: eine vereinfachte Schnittdarstellung der erfindungsgemässen Atemschlauch - Anschlussvorrichtung zur Erläuterung einer Verbindung eines neonatalen Atemschlauches mit einer neonatalen Atemvorrichtung,
- Fig. 3: eine vereinfachte Schnittdarstellung der erfindungsgemässen Atemschlauch - Anschlussvorrichtung zur Erläuterung einer Verbindung eines neonatalen Atemschlauches mit einer Atemvorrichtung für Erwachsene und
- Fig. 4: eine vereinfachte Schnittdarstellung der erfindungsgemässen Atemschlauch - Anschlussvorrichtung in einer zweiten Ausführung mit einem Transponder.

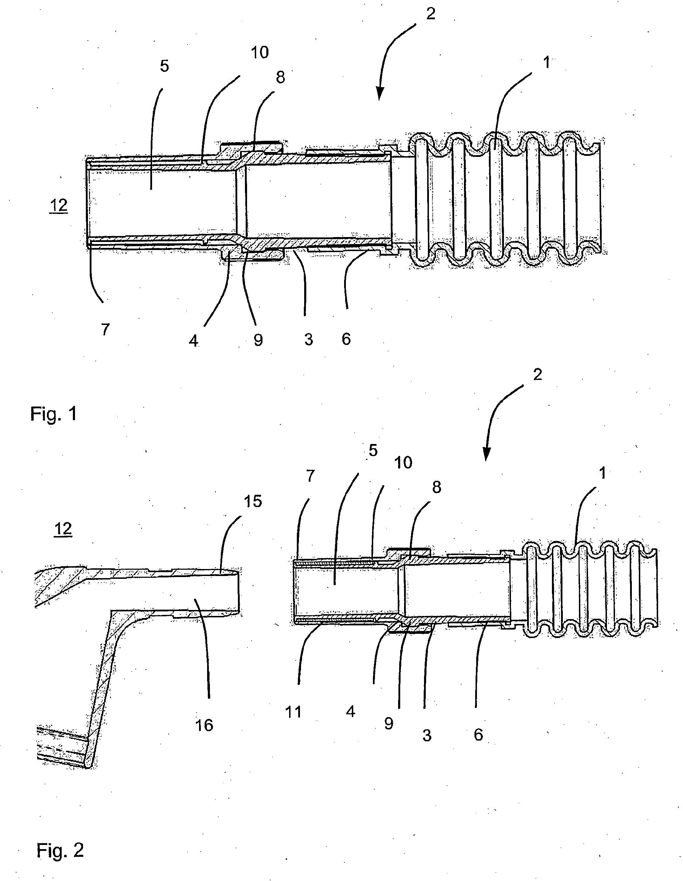

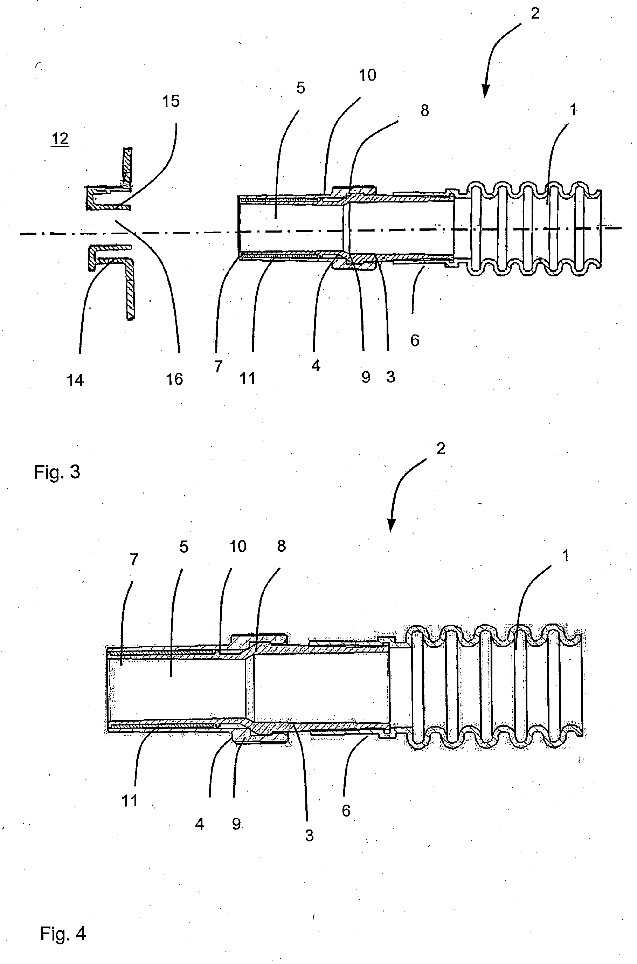

Der in Fig. 1 gezeigte Atemschlauch 1 ist in seinem Endbereich mit einer Atemschlauch - Anschlussvorrichtung 2 versehen, die einen Grundkörper 3 und einen zu einer Atemvorrichtung 12 gerichteten Teil des Grundkörpers 3 umschließenden Hüllkörper 4 aufweist. Im atemschlauchseitigen Bereich des Grundköpers 3 ist ein Atemschlauch - Befestigungsabschnitt 6 gebildet, in welchem der Atemschlauch 1 fixiert ist. Der Grundkörper 3 weist einen Atemgasdurchgangskanal 5 auf, dessen Durchgangsquerschnitt im Wesentlichen dem Durchgangsquerschnitt des an den Atemschlauch - Befestigungsabschnitt 6 angeschlossenen Atemschlauches 1 entspricht. Sowohl die Außenfläche des Grundkörpers 3 als auch die Innenwandung des Hüllkörpers 4 der in Fig. 1 dargestellte Atemschlauch - Anschlussvorrichtung 2 sind zylindrisch ausgebildet. In einem mittleren Bereich des Grundkörpers 3 ist an dessen Außenseite eine Umfangswulst 8 vorgesehen, über die ein unter mechanischen Gesichtspunkten günstiger Formschluss mit einer komplementären, an der Innenwandung des Hüllkörpers 4 befindlichen Umfangsnut 9, erreicht wird. Um ein Verdrehen des Hüllkörpers 4 gegenüber dem Grundkörper 3 bei einem Verbinden oder Lösen der Atemschlauch - Anschlussvorrichtung 2 mit oder von einer Atemvorrichtung 12 zu verhindern, ist eine senkrecht zu einer Außenfläche des Grundkörpers 3 gerichtete Nase vorgesehen, die in eine senkrecht zu der Außenseite des Hüllkörpers 4 gerichtete komplementäre Aussparung eingreift. Ein Einrasten der Nase des Grundkörpers 3 in die Aussparung des Hüllkörpers 4 signalisiert dabei eine exakte Verbindung von Grundkörper 3 und Hüllkörper 4. Der Grundkörper 3 und der Hüllkörper 4 sind vorzugsweise, aufgrund der guten elastischen Eigenschaften, aus Polypropylen (PP) ausgeführt. Es ist aber auch möglich, den Grundkörper 3 und Hüllkörper 4 aus anderen geeigneten Materialien zu fertigen. In den Figuren 2 und 3 sind zum Zwecke der Erläuterung der Verbindung von der erfindungsgemässen Atemschlauch - Anschlussvorrichtung 2 mit einer Atemvorrichtung 12 eine atemvorrichtungsseitige Anschlussstruktur 16 dargestellt, die im Wesentlichen komplementär zu dem von dem Grundkörper 3 und dem Hüllkörper 4 ausgebildeten Kopplungsabschnitt 7 ausgebildet ist. Dabei zeigt Fig. 2 eine Atemschlauch - Anschlussvorrichtung 2 zur Verbindung mit einer neonatalen Atemvorrichtung 12. Eine neonatale Atemvorrichtung 12 ist im Allgemeinen mit einer männlichen Anschlussstruktur 16 ausgeführt, die einen Durchmesser von 11 Millimetern aufweist. Eine Atemvorrichtung 12 für Erwachsene, wie sie in Fig. 3 dargestellt ist, weist hingegen üblicherweise eine männliche und eine weibliche Anschlussstruktur 16 auf, wobei die männliche Anschlussstruktur 16 einen Durchmesser von 15 Millimetern und die weibliche Anschlussstruktur 16 einen Durchmesser von 11 Millimetern besitzt. Die erfindungsgemässe Atemschlauch - Anschlussvorrichtung 2 kann in vorteilhafter Weise eine Verbindung eines neonatalen Atemschlauches 1 mit einer neonatale Atemvorrichtung 12 als auch mit einer Atemvorrichtung 12 für Erwachsene realisieren. Bei der Verbindung der Atemschlauch - Anschlussvorrichtung 2 eines neonatalen Atemschlauches 1 an eine in Fig. 2 schematisch dargestellte Anschlussstruktur 16 einer neonatalen Atemvorrichtung 12 gelangt der durch das Bezugszeichen 7 gekennzeichnete Kopplungsabschnitt der Atemschlauch-Anschlussvorrichtung 2 mit einem Zapfenabschnitt 15 der Anschlussstruktur 16 der neonatalen Atemvorrichtung 12 in Eingriff. Der Kopplungsabschnitt 7 wird von der Innenwandung des Grundkörpers 3, dem Atemgasdurchgangskanal 5, gebildet. Ein zu der Atemvorrichtung 12 gerichteter Teil des Atemgasdurchgangskanals 5 des Grundkörpers 3 ist in vorteilhafter Weise konusförmig ausgebildet. Der Zapfenabschnitt 15 der Anschlussstruktur 16, der in Fig. 2 dargestellten neonatalen Atemvorrichtung 12 weist eine dem Koppelabschnitt 7 komplementäre Form auf.

Bei der Verbindung der Atemschlauch - Anschlussvorrichtung 2 eines neonatalen Atemschlauches 1 an eine, in Fig. 3 schematisch dargestellte, Anschlussstruktur 16 der Atemvorrichtung 12 für Erwachsene, gelangt der Kopplungsabschnitt 7 der Atemschlauch - Anschlussvorrichtung 2 mit einer Ausnehmung 14 und dem Zapfenabschnitt 15 der Anschlussstruktur 16 der Atemvorrichtung 12 für Erwachsene in Eingriff. Der Kopplungsabschnitt 7 wird von der Innenwandung des Grundkörpers 3 und der Außenfläche des Hüllkörpers 4 gebildet. Somit lässt sich mit der erfindungsgemässen Atemschlauch - Anschlussvorrichtung 2 ein neonataler Atemschlauch 1 sowohl mit der Anschlussstruktur 16 der neonatalen Atemvorrichtung 12 (dargestellt in Fig. 2) als auch mit der Anschlussstruktur 16 einer Atemvorrichtung 12 für Erwachsene (dargestellt in Fig. 3) verbinden. Die Außenfläche des Hüllkörpers 4 ist, wie in Fig. 3 gezeigt, in vorteilhafter Weise konusförmig ausgebildet und wirkt mit der konusförmigen Ausnehmung 14 der Anschlussstruktur 16 der Atemvorrichtung 12 für Erwachsene zusammen.

In Fig. 4 ist eine ganz besonders bevorzugte Ausführung der Atemschlauch-Anschlussvorrichtung 2 gezeigt. Zwischen der Außenfläche des Grundkörpers 3 und der Innenwandung des Hüllkörpers 4 ist ein Transponder 11 vorgesehen. Der Transponder 11 dient der drahtlosen Sendung und des drahtlosen Empfangs von Daten und ist zudem mit einer Funktion zur Speicherung der Daten ausgestattet. Die auf der Außenfläche des Grundkörpers 3 vorgesehene Umfangswulst 8 dient als Positionierungshilfe beim Aufbringen des Transponders 11 auf dem Grundkörper 3. Damit wird gewährleistet, dass der Transponder 11 eine reproduzierbare Position aufweist und sehr nah an einem Ende einer zu der Atemvorrichtung 12 weisenden Seite des Grundkörpers 3 vorgesehen ist. Dies ermöglicht die Verwendung von passiven Transpondern 11, beispielsweise eines RFID - Transponders 11 mit einer Reichweite von zirka 18 Millimetern. Der Empfang und die Sendung von Daten sind somit optimal gewährleistet. Der Transponder 11 der erfindungsgemässen Atemschlauch - Anschlussvorrichtung 2 der Fig. 4 ermöglicht durch eine Speicherfunktion von Daten in vorteilhafter Weise eine Ablage von Atemparametem, wie beispielsweise eine Atemfrequenz, einen Atemhub, einen Atemdruck und / oder eine Form der Beatmung. Wird nur der Atemschlauch 1 von einer neonatale Atemvorrichtung 12 entkoppelt und an eine Atemvorrichtung 12 für Erwachsene gekoppelt, können die auf dem Transponder 11 hinterlegten Atemparameter der neonatalen Atemvorrichtung 12 auf die Atemvorrichtung 12 für Erwachsene übertragen werden, bzw. diese Atemparameter an der Atemvorrichtung 12 für Erwachsene eingestellt werden. Zudem kann ein Atemmode für Erwachsene gesperrt und nur ein Atemmode für Neonaten an der Atemvorrichtung 12 für Erwachsene freigegeben werden. Damit ist eine Einstellung von zu hohen Atemdrücken für Neonaten, wie sie für eine Beatmung von Erwachsenen erforderlich sind, ausgeschlossen. Weiterhin lassen sich personengebundene Daten, wie beispielsweise eine Patentenidentifikationsnummer, mit dem Transponder 11 der erfindungsgemässen Atemschlauch - Anschlussvorrichtung 2 speichern. Der Atemschlauch 1 verbleibt bei einem Wechsel des klinischen Bereiches und damit der Atemvorrichtung am Patienten. Die in dem Transponder 11 hinterlegten Daten sind somit mit dem Atemschlauch 1 verfügbar und ermöglichen bei einem Anschluss des Atemschlauches 1 an die Atemvorrichtung 12 für Erwachsene eine Übertragung der Daten an die Atemvorrichtung 12. Mittels einer Patientenidentifikationsnummer kann sich beispielsweise eine an ein Kliniknetzwerk angeschlossene Atemvorrichtung 12 auf die für den entsprechenden Patienten erforderlichen Atemparameter einstellen.

In einer weiteren Ausgestaltung der in der Fig. 4 gezeigten Atemschlauch-Anschlussvorrichtung 2 werden Kenngrössen für eine Gewährleistung einer einwandfreien Funktion des Atemschlauches 1 auf den Transponder 11 übertragen. Beispielsweise wird die Zeit der Nutzung des Atemschlauches 1 an der jeweiligen Atemvorrichtung 12 gemessen und jeweils auf den Transponder 11 übermittelt. Somit kann die gesamte Lebensdauer des Atemschlauches 1 auf dem Transponder 11 hinterlegt werden und nach dem Erreichen einer Zeitdauer, die einen Ablauf für die Gewährleistung der einwandfreien Funktion des Atemschlauches 1 definiert, kann eine entsprechende Information dem Anwender übermittelt werden.

Ein bevorzugtes Verfahren zur Herstellung der erfindungsgemäßen Atemschlauch - Anschlussvorrichtung 2 wird im Folgenden anhand des in Fig. 4 dargestellten Ausführungsbeispiels erläutert. In einem ersten Verfahrensschritt wird der Grundkörper 3 mittels eines Spritzgussverfahrens hergestellt. Der Grundkörper 3 weist dabei eine von der Außenfläche herausragende Umlaufwulst 8 auf, die zur Positionierung eines in einem weiteren Verfahrensschritt auf die Außenfläche des Grundkörpers 3 aufzubringenden Transponders 11 dient. Der Transponder 11 wird dabei auf einem zu der Atemvorrichtung 12 weisenden Teil der Außenfläche des Grundkörpers 3 aufgebracht. Danach oder parallel wird der Hüllkörper 4 mittels eines Spritzgussverfahrens hergestellt. In einem weiteren Verfahrensschritt wird der Grundkörper 3 mit dem Hüllkörper 4 formschlüssig verbunden.

### BEZUGSZEICHENLISTE

- 1: Atemschlauch
- 2: Atemschlauch - Anschlussvorrichtung
- 3: Grundkörper
- 4: Hüllkörper
- 5: Atemgasdurchgangskanal
- 6: Atemschlauch - Befestigungsabschnitt
- 7: Kopplungsabschnitt
- 8: Umfangswulst
- 9: Umfangsnut
- 10: Verdrehsicherung
- 11: Transponder
- 12: Atemvorrichtung
- 14: Ausnehmung
- 15: Zapfenabschnitt
- 16: Anschlussstruktur

## Patentansprüche

1. Atemschlauch - Anschlussvorrichtung zur Verbindung eines Atemschlauches mit einer Atemvorrichtung, wobei die Atemschlauch-Anschlussvorrichtung aufweist:
- einen Grundkörper (3) mit einem in dem Grundkörper (3) ausgebildeten Atemgasdurchgangskanal (5) und einem Atemschlauch-Befestigungsabschnitt (6) zur Fixierung eines Atemschlauches (1) und
- einen Hüllkörper (4),
**dadurch gekennzeichnet, dass** der Hüllkörper (4) mit dem Grundkörper (3) in der Weise formschlüssig in Eingriff bringbar ist, dass er dabei zumindest einen im Einsatz zu einer Atemvorrichtung (12) gerichteten Teil des Grundkörpers (3) koaxialförmig umschließt und der Grundkörper (3) und der Hüllkörper (4) einen Kopplungsabschnitt (7) ausbilden, in dem der Grundkörper (3) und der Hüllkörper (4) jeweils zur Verbindung der Atemschlauch-Anschlussvorrichtung (2) mit verschiedenen Atemvorrichtungen (12) angepasst sind.

2. Atemschlauch - Anschlussvorrichtung nach Anspruch 1, **dadurch gekennzeichnet, dass** der Durchgangsquerschnitt des Atemgasdurchgangskanals (5) im Wesentlichen dem Durchgangsquerschnitt des Atemschlauchs (1) entspricht.

3. Atemschlauch - Anschlussvorrichtung nach einem der vorhergehenden Ansprüche, **dadurch gekennzeichnet, dass** ein zu der Atemvorrichtung (12) gerichtete Teil des Atemgasdurchgangskanals (5) konusförmig verläuft und eine Außenfläche des Grundkörpers (3) zylindrisch ausgebildet ist.

4. Atemschlauch - Anschlussvorrichtung nach einem der vorhergehenden Ansprüche, **dadurch gekennzeichnet, dass** eine Innenwandung des Hüllkörpers (4) zylindrisch ausgebildet ist und eine Außenfläche konisch verläuft.

5. Atemschlauch - Anschlussvorrichtung nach einem der vorhergehenden Ansprüche, **dadurch gekennzeichnet, dass** ein Formschluss von Grund- und Hüllkörper (3, 4) durch einen Eingriff einer an der Außenfläche des Grundkörpers (3) vorgesehenen Umfangswulst (8) in eine komplementäre an der Innenwandung des Hüllkörpers (4) vorgesehenen Umfangsnut (9) erfolgt.

6. Atemschlauch - Anschlussvorrichtung nach einem der vorhergehenden Ansprüche, **dadurch gekennzeichnet, dass** Mittel (10) vorgesehen sind, die ein Verdrehen des Hüllkörpers (4) in Bezug zu dem Grundkörper (3) verhindern.

7. Atemschlauch - Anschlussvorrichtung nach Anspruch 6, **dadurch gekennzeichnet, dass** der Grundkörper (3) eine senkrecht zu der Außenfläche gerichtete Nase und der Hüllkörper (4) eine senkrecht zu der Innenwandung gerichtete komplementäre Aussparung aufweist.

8. Atemschlauch - Anschlussvorrichtung nach einem der vorhergehenden Ansprüche, **dadurch gekennzeichnet, dass** der Grundkörper (3) und der Hüllkörper (4) aus Polypropylen besteht.

9. Atemschlauch - Anschlussvorrichtung nach einem der vorhergehenden Ansprüche, **dadurch gekennzeichnet, dass** ein Transponder (11) zwischen dem Grundkörper (3) und dem Hüllkörper (4) vorgesehen ist.

10. Atemschlauch - Anschlussvorrichtung nach Anspruch 9, **dadurch gekennzeichnet, dass** der Transponder (11) den Grundkörper (3) koaxialförmig umschließt.

11. Atemschlauch - Anschlussvorrichtung nach einem der Ansprüche 9 oder 10, **dadurch gekennzeichnet, dass** die Außenfläche des Grundkörpers (3) Hilfsmittel zur Positionierung des Transponders (11) auf dem Grundkörper (3) aufweist.

12. Atemschlauch - Anschlussvorrichtung nach einem der Ansprüche 9 bis 11, **dadurch gekennzeichnet, dass** der Transponder (11) ein Speicherelement enthält.

13. Atemschlauch - Anschlussvorrichtung nach einem der Ansprüche 9 bis 12, **dadurch gekennzeichnet, dass** der Transponder (11) an einer zu der Atemvorrichtung (12) weisenden Seite des Grundkörpers (3) vorgesehen ist.

14. Verwendung einer Atemschlauch - Anschlussvorrichtung nach einem der vorhergehenden Ansprüche zur Verbindung eines Atemschlauchs (1) mit einer ersten oder einer zweiten Atemvorrichtung (12), wobei die erste und die zweite Atemvorrichtung (12) eine unterschiedliche Anschlussstruktur (16) aufweist.

15. Verwendung einer Atemschlauch - Anschlussvorrichtung nach einem der Ansprüche 9 bis 14, **dadurch gekennzeichnet, dass** Parameter der ersten Atemvorrichtung (12) auf die zweite Atemvorrichtung (12) übertragbar sind.

## Claims

1. A breathing tube connecting device for connecting a breathing tube to a respirator, the breathing tube connecting device comprising:
- a base body (3) with a breathing gas through duct (5) embodied in the base body (3) and a breathing tube fastening section (6) for fixing the breathing tube (1) and
- a jacket body (4),
**characterized in that** the jacket body (4) can be meshed with the base body (3) in a positive-locking manner, **in that** it coaxially encloses at least a portion of the base body (3) directed to a respirator (12) during operation, and **in that** the base body (3) and the jacket body (4) are forming a coupling section (7), wherein the base body (3) and the jacket body (4) are each arranged for connection of the breathing tube connecting device (2) to different respirators (12).

2. The breathing tube connecting device according to claim 1, **characterized in that** the through cross section of the breathing gas through duct (5) essentially corresponds to the through cross section of the breathing tube (1).

3. The breathing tube connecting device according to any of the preceding claims, **characterized in that** a part of the breathing gas through duct directed toward the respirator (12) extends in a cone-shaped manner, and an outer surface of the base body (3) has a cylindrical design.

4. The breathing tube connecting device according to any of the preceding claims, **characterized in that** an inner wall of the jacket body (4) has a cylindrical design and an outer surface extends in a cone-like manner.

5. The breathing tube connecting device according to any of the preceding claims, **characterized in that** a positive-locking connection of base and jacket bodies (3, 4) is formed by means of a meshing of a circumferential bearing (8) with a complementary circumferential groove (9) provided on the inner wall of the jacket body (4).

6. The breathing tube connecting device according to any of the preceding claims, **characterized in that** means (10) are provided which prevent rotation of the jacket body (4) in relation to the base body (3).

7. The breathing tube connecting device according to claim 6, **characterized in that** the base body (3) has a lug directed at a right angle to the outer surface, and **in that** the jacket body (4) has a complementary cutout directed at a right angle to the inner wall.

8. The breathing tube connecting device according to any of the preceding claims, **characterized in that** the base body (3) and the jacket body (4) consist of polypropylene.

9. The breathing tube connecting device according to any of the preceding claims, **characterized in that** a transponder (11) is provided between the base body (3) and the jacket body (4).

10. The breathing tube connecting device according to claim 9, **characterized in that** the transponder (11) encloses the base body (3) in a coaxial manner.

11. The breathing tube connecting device according to any of the claims 9 or 10, **characterized in that** the outer surface of the base body (3) comprises auxiliary means for positioning of the transponder (11) on the base body (3).

12. The breathing tube connecting device according to any of the claims 9 to 11, **characterized in that** the transponder (11) contains a storage element.

13. The breathing tube connecting device according to any of the claims 9 to 12, **characterized in that** the transponder (11) is provided on a side of the base body (3) pointing toward the respirator (12).

14. Use of a breathing tube connecting device according to any of the preceding claims for connecting a breathing tube (1) to a first or a second respirator (12), wherein the first and the second respirator (12) comprise different connecting structures (16).

15. Use of a breathing tube connecting device according to any of the claims 9 to 14, **characterized in that** parameters of the first respirator (12) can be transferred to the second respirator (12)

## Revendications

1. Dispositif de raccordement de tube respiratoire servant à relier un tube respiratoire à un dispositif respiratoire, lequel dispositif de raccordement de tube respiratoire comporte :
- un corps de base (3) avec un canal de passage de gaz respiratoire (5) formé dans le corps de base (3) et une portion de fixation de tube respiratoire (6) servant à fixer un tube respiratoire (1) et
- un corps enveloppant (4)
**caractérisé en ce que** le corps enveloppant (4) peut être amené en engagement par complémentarité de formes avec le corps de base (3) de façon à entourer coaxialement au moins une partie du corps de base (3) dirigée, en utilisation, vers un dispositif respiratoire (12), et **en ce que** le corps de base (3) et le corps enveloppant (4) forment une portion d'accouplement (7) dans laquelle le corps de base (3) et le corps enveloppant (4) sont adaptés chacun pour relier le dispositif de raccordement de tube respiratoire (2) à différents dispositifs respiratoires (12).

2. Dispositif de raccordement de tube respiratoire selon la revendication 1, **caractérisé en ce que** la section de passage du canal de passage de gaz respiratoire (5) correspond sensiblement à la section de passage du tube respiratoire (1).

3. Dispositif de raccordement de tube respiratoire selon une des revendications précédentes, **caractérisé en ce qu'**une partie du canal de passage de gaz respiratoire (5) dirigée vers le dispositif respiratoire (12) s'étend en forme de cône, et une surface extérieure du corps de base (3) est cylindrique.

4. Dispositif de raccordement de tube respiratoire selon une des revendications précédentes, **caractérisé en ce qu'**une paroi intérieure du corps enveloppant (4) est cylindrique et une surface extérieure s'étend en forme de cône.

5. Dispositif de raccordement de tube respiratoire selon une des revendications précédentes, **caractérisé en ce qu'**un engagement par complémentarité de formes des corps de base et enveloppant (3, 4) est réalisé par un engagement d'un bourrelet périphérique (8), prévu sur la surface extérieure du corps de base (3), dans une gorge périphérique (9) complémentaire prévue sur la paroi intérieure du corps enveloppant (4).

6. Dispositif de raccordement de tube respiratoire selon une des revendications précédentes, **caractérisé en ce que** des moyens (10) qui empêchent une rotation du corps enveloppant (4) par rapport au corps de base (3) sont prévus.

7. Dispositif de raccordement de tube respiratoire selon la revendication 6, **caractérisé en ce que** le corps de base (3) comporte un ergot dirigé perpendiculairement à la surface extérieure, et le corps enveloppant (4) comporte un évidement complémentaire dirigé perpendiculairement à la paroi intérieure.

8. Dispositif de raccordement de tube respiratoire selon une des revendications précédentes, **caractérisé en ce que** le corps de base (3) et le corps enveloppant (4) sont en polypropylène.

9. Dispositif de raccordement de tube respiratoire selon une des revendications précédentes, **caractérisé en ce qu'**un transpondeur (11) est prévu entre le corps de base (3) et le corps enveloppant (4).

10. Dispositif de raccordement de tube respiratoire selon la revendication 9, **caractérisé en ce que** le transpondeur (11) entoure coaxialement le corps de base (3).

11. Dispositif de raccordement de tube respiratoire selon une des revendications 9 ou 10, **caractérisé en ce que** la surface extérieure du corps de base (3) comporte des moyens auxiliaires pour positionner le transpondeur (11) sur le corps de base (3).

12. Dispositif de raccordement de tube respiratoire selon une des revendications 9 à 11, **caractérisé en ce que** le transpondeur (11) contient un élément de mémoire.

13. Dispositif de raccordement de tube respiratoire selon une des revendications 9 à 12, **caractérisé en ce que** le transpondeur (11) est prévu sur un côté du corps de base (3) dirigé vers le dispositif respiratoire (12).

14. Utilisation d'un dispositif de raccordement de tube respiratoire selon une des revendications précédentes pour relier un tube respiratoire (1) à un premier ou un deuxième dispositif respiratoire (12), les premier et deuxième dispositifs respiratoire (12) présentant une structure de raccordement (16) différente.

15. Utilisation d'un dispositif de raccordement de tube respiratoire selon une des revendications 9 à 14, **caractérisé en ce que** des paramètres du premier dispositif respiratoire (12) sont transposables au deuxième dispositif respiratoire (12).
